# EUROPÄISCHE PATENTANMELDUNG

(11) **EP 4 640 245 A1**
(43) Veröffentlichungstag der Anmeldung: **29.10.2025**
(21) Anmeldenummer: 24172415.2
(22) Anmeldetag: 25.04.2024
(51) Int. Cl.: A61L 15/22, A61L 15/46

(54) **WUNDKONTAKTSCHICHT MIT INFEKTIONSHEMMENDEN EIGENSCHAFTEN**

(71) Anmelder: Paul Hartmann AG, 89522 Heidenheim (DE)
(72) Erfinder: KETTEL, Markus, 89520 Heidenheim (DE); KARL, Michael Maximilian, 89522 Heidenheim (DE); VRANA, Nihal Engin, 6700 Strasbourg (FR); HATHROUBI, Skander, 6700 Strasbourg (FR)
(74) Vertreter: Paul Hartmann AG Patents & Licensing

(57) **Zusammenfassung**

Die vorliegende Erfindung betrifft eine Wundkontaktschicht umfassend ein netzförmiges Substrat, welches teilweise oder vollständig eine antimikrobielle Beschichtung aufweist. Die Beschichtung umfasst Hyaluronsäure und ein Polypeptid ausgewählt aus Polyarginin und / oder Polylysin. Darüber hinaus werden Verfahren zum Auftragen der Beschichtung auf die Wundkontaktschicht erläutert. Die Beschichtung zeichnet sich durch ihre gute Verträglichkeit und Zellkompatibilität bei gleichzeitig starker antiseptischer Wirkung aus.

## Beschreibung

### Technisches Gebiet der Erfindung

Die vorliegende Erfindung betrifft die Wundbehandlung, insbesondere die Behandlung schwerer, chronischer und/oder infizierter Wunden. Häufig ist in solchen Fällen der Heilungsprozesses gestört oder stagniert und es werden große Mengen an Exsudat von der Wunde abgeben.

### Hintergrund der Erfindung

Chronische Wunden stellen in der modernen Medizin nach wie vor ein Problem dar. Gerade bei älteren Menschen und Risikopatienten wie beispielsweise Diabetikern besteht ein erhöhtes Risiko, dass Verletzungen nicht oder nicht vollständig abheilen. Der Wundheilungsprozess ist in einem solchen Fall aus unterschiedlichen Gründen gestört und es besteht ein fortwährender Defekt der Hautbarriere. Dies geht zum einen mit einer Reduktion der Lebensqualität für die Betroffenen einher. Zum anderen erhöht sich mit fortwährender Dauer der Gewebeexposition zunehmend das Risiko einer Infektion. Tritt eine solche Infektion ein, verschlechtert sich die Prognose weiter. Da die körpereigenen Stoffwechselprozesse im Bereich der chronischen Wunde beeinträchtigt sind und eine reguläre Immunantwort nicht oder nicht vollständig erfolgt, kann es in der Folge zu einer Vermehrung der Erreger an der Infektionsstelle kommen. Im weiteren Verlauf bildet sich häufig ein Biofilm, bei dem bakterielle Erreger sich zu einer Lebensgemeinschaft vereinen, die erhöhte Resistenz gegenüber Bioziden und Antibiotika zeigt und daher die weitere Behandlung außerordentlich erschwert. Dieses Stadium erhöht die Wahrscheinlichkeit für weitere Komplikationen wie beispielsweise Nekrosenbildung oder Sepsis.

Aus dem Stand der Technik sind Wundauflagen mit antibiotischer Wirksamkeit bekannt. Diese beruhen in der Regel auf der Verwendung körperfremder antimikrobieller Wirkstoffe. Derartige Wundauflagen zeigen zwar eine Wirkung gegen pathogene Mikroorganismen, haben jedoch die nachteilige Eigenschaft auch körpereigene Zellen zu stressen. Mittels geeigneter *in vitro* Assays kann eine erhöhte Zytotoxizität für tierische Zellen nachgewiesen werden. Die betroffenen Zellen werden gestresst und ihre Vitalwerte sinken ab. Bei Testkonzentrationen, die den Realbedingungen nahekommen, stirbt für gewöhnlich ein Teil der im Assay befindlichen Zellen ab.

Die EP 1 755 569 B9 beschreibt eine Wundauflage mit Salbe, die zusätzlich ein antibakterielles Metall wie beispielsweise Silber enthält. Allerdings geht die Wirkung von Silber mit deutlich messbarer Zytotoxizität einher.

Die EP 3 452 118 B1 beschreibt antimikrobielle Beschichtungen, die Hyaluronsäure und ein Polypeptid enthalten, wobei die Säure und das Peptid nicht durchmischt vorliegen. Spezifische Ausgestaltungen von Wundauflagen werden nicht genannt.

Folglich besteht ein Bedarf nach einem spezifisch ausgestalteten Mittel zur Wundabdeckung, das ausreichende antimikrobielle Wirksamkeit zeigt, nicht cytotoxisch für menschliche Zellen ist und zudem atraumatisch und schmerzfrei entfernt werden kann. Um dem klinischen Alltag gerecht zu werden, sollte das Mittel der Wahl zudem unmittelbar einsetzbar, bei längerer Lagerung über eine ausreichende Stabilität verfügen und beständig gegenüber möglichen Temperaturschwankungen sein.

### Zusammenfassung der Erfindung

Wundkontaktschicht umfassend ein netzförmiges Substrat, welches teilweise oder vollständig eine antimikrobielle Beschichtung aufweist, welche Hyaluronsäure und Polypeptide ausgewählt aus Polyarginin oder Polylysin oder einer Mischung aus Polyarginin und Polylysin umfasst.

Die erfindungsgemäße Wundkontaktschicht hat hervorragende atraumatische Eigenschaften, d.h. sie verbindet sich nicht mit der Wunde oder Wundbestandteilen. Weder kann Gewebe in die Wundkontaktschicht einwachsen, noch kann die Wundkontaktschicht am Wundbett ankleben. Der für gewöhnlich notwendige Einsatz von Salbe oder Silikongel zum Erreichen der atraumatischen Eigenschaften ist bei der vorliegenden Erfindung nicht notwendig, da diese Rolle von der antimikrobiellen Beschichtung erfüllt wird. Schließlich ist die Beschichtung aufgrund ihrer antimikrobiellen Inhaltsstoffe gegen humanpathogene Bakterien wirksam. Somit werden durch die Beschichtung gleich zwei vorteilhafte Wirkungen erzielt. Zudem kann die Wundkontaktschicht nach dem Anbringen an den Patienten durch einen passenden Sekundärverband überlagert und fixiert werden. Die Art des Sekundärverbands kann dabei je nach Wundart spezifisch ausgewählt werden, um die bestmögliche Versorgung zu gewährleisten.

Die erfindungsgemäße Wundkontaktschicht kann auch dann zum Einsatz kommen, wenn sich in der zu behandelnden Wunde antibiotikaresistente Bakterien befinden. Während

Antibiotika üblicherweise organische Verbindungen sind, die von resistenten Bakterien beispielsweise abgebaut werden, ist ein derartiges Auftreten von Resistenzen im Hinblick auf die antimikrobielle Beschichtung der vorliegenden Erfindung nicht beobachtet worden.

Die erfindungsgemäße Wundkontaktschicht kann, auch bei nicht infizierten Wunden, wundheilungsfördernde Eigenschaften aufweisen. Dies ergibt sich durch die feuchtigkeitsregulierenden Eigenschaften der Hyaluronsäure, die auch *in vivo* im Bereich der extrazellulären Matrix zu finden ist.

Nachfolgend wird erläutert, wie die Wundkontaktschicht strukturell und chemisch beschaffen sein kann, um in der Praxis den größtmöglichen Nutzen zu erbringen.

### Detaillierte Beschreibung der Erfindung

Der Begriff "medizinisch akzeptables Material" im Sinne der Erfindung ist eine ungiftige, fusselfreie und stabile Substanz, die sich unter Normalbedingungen weder in polaren noch in unpolaren Verbindungen löst und weder von den Absonderungen tierischer noch bakterieller Zellen in nennenswertem Ausmaß abgebaut oder verflüssigt werden kann.

Der Begriff "netzförmiges Substrat" meint, dass das Substrat in Form eines Netzes mit Stegen und zwischen den Stegen liegenden Öffnungen vorliegt. Insbesondere sind Öffnungen vorhanden, die sich von einer Seite des netzförmigen Substrates zur gegenüberliegenden Seite erstrecken. Unter einer Öffnung im Sinne der vorliegenden Erfindung ist somit ein durch das Substrat hindurchgehender Hohlraum zu verstehen, der geeignet ist, eine Flüssigkeit wie zum Beispiel Wundexsudat von einer Oberfläche des Substrats zur gegenüberliegenden Oberfläche des Substrats passieren zu lassen.

Eine netzförmige Struktur im Sinne der vorliegenden Erfindung kann beispielsweise durch ein Gitter, ein Gewebe, ein Gewirke - insbesondere ein Tüllgewebe - , ein perforiertes Vlies oder durch eine perforierte Folienlage erzeugt werden.

Der Ausdruck "Kolonie bildende Einheit" (CFU) meint eine einzelne teilungsfähige Zelle eines Einzellers, insbesondere eines humanpathogenen Einzellers oder Bakteriums.

"Beschichtet" meint, dass eine Oberfläche eines Festkörpers mittels einer Substanz, die sich von der Struktur des Festkörpers unterscheidet, zumindest teilweise bedeckt bzw. überlagert wird.

Der Ausdruck "konfiguriert zum Auflegen auf eine Wunde" meint, zum Auflegen auf eine Wunde zu einem therapeutischen Zweck im Sinne einer Wundbehandlung vorgesehen und geeignet.

Der Ausdruck "atraumatisch" meint, dass ein Wundversorgungsprodukt sich nicht mit der Wunde fest verbindet, also beispielsweise nicht in der Wunde eintrocknet oder mit dieser verwächst und dass eine schmerzfreie Entfernung des Produktes ohne Störung des Heilungsprozesses möglich ist.

Der Ausdruck "antimikrobielle Inhaltsstoffe" meint Hyaluronsäure und ein oder mehrere Polypeptide, bei denen es sich um Polyarginin und / oder Polylysin und / oder Polyornithin handelt.

Die Ausdrücke "Aminosäure" und "Aminosäuren" beziehen sich vorliegend, sofern nicht anders angegeben, auf die Verbindungen Arginin, Lysin und / oder Ornithin, welche allesamt zu den positiv geladenen Aminosäuren zählen. Dies schließt insbesondere die L-Enantiomere der genannten Aminosäuren ein.

Die Ausdrücke "Polypeptid" und "antimikrobielles Polypeptid" meinen vorliegend Polyarginin, Polylysin und / oder Polyornithin und beziehen sich auf Peptidverbindungen, die mindestens 11, bevorzugt mindestens 20, besonders bevorzugt mindestens 30 Untereinheiten in Form von verbundenen Aminosäuren umfassen. Im Rahmen der Erfindung können die Aminosäuren innerhalb eines solchen Polypeptids allesamt identisch sein (selbe Aminosäure). Alternativ können Mischungen aus zwei oder drei der zuvor genannten Aminosäuren innerhalb eines Polypeptids vorliegen. Alle derartigen Polypeptide haben eine positive Nettoladung. Dem Fachmann ist bekannt, dass sich für die Aminosäuren am C-terminalen sowie N-terminalen Ende naturgemäß strukturelle Unterschiede ergeben, da diese Positionen die jeweiligen Kettenenden darstellen.

Der Ausdruck "Polylysin" umfasst die stereochemischen Varianten α-Poly-L-Lysin und ε-Poly-L-Lysin.

Die Ausdrücke "abwechseln" und "abwechselnd" meinen, dass auf eine erste Lage enthaltend Hyaluronsäure eine zweite Lage enthaltend das Polypeptid oder die Polypeptide folgt. Eine optionale dritte Lage würde folglich wieder Hyaluronsäure enthalten. Auf diese Wiese wechseln sich die Lagen in ihrer Nettoladung ab und bilden aufgrund ihrer Anziehungskräfte eine stabile Beschichtung aus. Alternativ kann in diesem Sinne natürlich auch auf eine erste Lage enthaltend das Polypeptid oder die Polypeptide eine zweite Lage enthaltend die Hyaluronsäure usw. folgen.

Der Ausdruck "proximal" bedeutet, dass ein Element im Einsatz zur Wunde oder Haut hin ausgerichtet ist.

Der Ausdruck "distal" bedeutet, dass ein Element im Einsatz von der Wunde oder Haut weg zeigt.

Die vorliegende Erfindung betrifft eine Wundkontaktschicht. Diese Wundkontaktschicht kann allein verwendet werden oder nach dem Anbringen auf eine Wunde bei Bedarf durch einen absorbierenden Sekundärverband überlagert werden oder mittels Sekundärverband, Klebefolie, Klebestreifen oder anderer Fixiermittel am Ort der Wunde befestigt werden. Bei Bedarf, z.B. wenn der Sekundärverband mit Wundexsudat gesättigt ist oder wenn sich die angebrachte Klebebefestigung löst, können Sekundärverband oder Klebemittel ersetzt werden, ohne die Wundkontaktschicht von der Wunde lösen zu müssen. Die Wundruhe bleibt auf diese Weise erhalten und ein unerwünschtes Eintrocknen des Sekundärverbands in der Wunde wird vermieden.

Vorteilhaft ist, dass die Wundkontaktschicht mittels der enthaltenen Beschichtung an der Haut atraumatisch anhaftet. Diese leichte Initialhaftung erleichtert das Auflegen auf die Wunde. In den meisten Fällen muss die Wundkontaktschicht nicht bis zur Applikation eines Fixiermittels an der Wundstelle festgehalten werden, sondern hält zunächst von allein, so dass dem Anwender beide Hände für die Vorbereitung des Sekundärverbands zur Verfügung stehen. Ein zusätzlicher Sekundärverband bietet den Vorteil, dass ein Verrutschen der Wundkontaktschicht verhindert wird und gewährleistet einen fortwährenden Kontakt zwischen Beschichtung und Wunde, so dass die antimikrobiellen Inhaltsstoffe an der Wunde wirken können.

Die erfindungsgemäße Wundkontaktschicht eignet sich für akute und blutende Wunden, chronische Wunden, nässende Wunden, Brandwunden (bis zu Verbrennungen 2. Grades) sowie Wunden, die nekrotisches Gewebe oder Fibrinbeläge enthalten. In der Praxis treten häufig Mischformen dieser Wunden auf, z.B. infizierte, teilnekrotische, chronische Wunden. In solchen Fällen zeigen sich die vorteilhaften Eigenschaften der erfindungsgemäßen Wundkontaktschicht besonders deutlich, da mit nur einem Produkt eine Vielzahl unterschiedlicher pathologischer Wundmilieus behandelt wird.

Die Wundkontaktschicht umfasst mindestens ein netzförmiges Substrat und eine antimikrobielle, insbesondere antibakterielle Beschichtung, die antimikrobielle Inhaltsstoffe enthält. Weitere Bestandteile können bei Bedarf hinzugefügt werden, um die Wundkontaktschicht an den vorgesehenen Einsatzzweck anzupassen. Dies wird an anderer Stelle näher erläutert.

Im Rahmen der vorliegenden Erfindung kann vorgesehen sein, dass sich die Beschichtung ausschließlich auf der ersten Seite des Substrats befindet und damit auf derjenigen Seite, welche der Wunde zugewandt ist. Auf diese Weise ist die für den Wundkontakt vorgesehene Seite eindeutig vom Anwender erkennbar und bedarf keiner weiteren Kennzeichnung. Außerdem hat diese Ausführung den ökonomischen Vorteil, dass Beschichtungsmaterial gespart wird, ohne dass die Versorgung der Wunde darunter leidet.

Das in der erfindungsgemäßen Wundkontaktschicht enthaltene Substrat ist ein flexibler und elastischer Festkörper, der sich der Form der Körper- oder Wundoberfläche anpassen kann. Das Substrat besteht aus einem festen, unlöslichen, medizinisch akzeptablen Material oder aus daraus hergestellten Fasern, wobei das Material vorzugsweise über eine Kristallgitterstruktur verfügt. Alternativ kann das Material als teilkristalliner oder amorpher Festkörper vorliegen (z.B. als amorpher Thermoplast wie Polyvinylchlorid). Bei dem Material kann es sich um ein Polymer- oder um eine Polymermischung handeln, insbesondere aus einem thermoplastischen Polymer oder einer Mischung thermoplastischer Polymere. Bevorzugt ist das Substrat flach bzw. planar, so dass es eine im Wesentlichen einheitliche Dicke aufweist und weder die erste noch die zweite Seite Erhebungen aufweisen. Geringfügige produktionsbedingte Toleranzen sind hierbei zu vernachlässigen, so dass im Sinne der Erfindung eine einheitliche Dicke auch bei Abweichungen von +/- 5 % als gegeben gelten soll. Ein flach ausgebildetes Substrat kann in der Aufsicht eine rechteckige, quadratische, ovale oder runde Form haben, wobei ovale oder runde Formen sich besonders für Wunden an Gelenken eignen und rechteckige oder quadratische Formen sich einfacher verpacken lassen und den Lagerplatz effizienter nutzen. Die Wundkontaktschicht kann die Form des Substrats haben, was jedoch nicht zwingend erforderlich ist.

Das Substrat hat eine erste Seite, welche in Benutzung der Wunde zugewandt ist (proximale Ausrichtung) und eine der ersten Seite gegenüberliegende zweite Seite, welche in Benutzung von der Wunde abgewandt ist (distale Ausrichtung).

Das Substrat ist netzförmig ausgestaltet. Die netzförmige Ausgestaltung erlaubt den Durchtritt von Wundexsudat ausgehend von der ersten Seite des Substrats in Richtung der zweiten Seite des Substrats. Daneben verleiht die Netzform dem Substrat eine ausgesprochene Flexibilität, da die Materialspannung beim Auflegen auf eine Wunde und der damit einhergehenden (reversiblen) Verformung minimiert wird. Zu guter Letzt wird durch das Netz die Kontaktfläche zur Wunde und damit die Grenzflächenspannung reduziert, so dass sich das Substrat besonders schonend von der Wunde ablösen lässt.

Eine mögliche Netzform ist die Form eines Gitters, bei welchem das Substrat Stege in Form von Längs- und Querstreben enthält, welche im Wesentlichen rechtwinklig zueinander angeordnet sind, so dass die Öffnungen im Substrat die Form von Rechtecken einnehmen. Gemäß einer bevorzugten Ausführungsform liegt die netzförmige Ausprägung in Form eines gleichförmigen Musters vor, so dass alle Öffnungen oder Poren im Wesentlichen dieselbe räumliche Ausdehnung und denselben Abstand zueinander haben. Der hieraus resultierende Vorteil ist, dass über die gesamte Fläche der ersten und zweiten Seite im Wesentlichen gleichbleibende Parameter und Kräfte vorherrschen und die antimikrobiellen Inhaltsstoffe folglich gleichmäßig an allen Positionen einer Wunde wirken können.

Bevorzugt enthält das Substrat Fasern. Das Substrat kann ausschließlich aus Fasern bestehen. Diese Fasern können die folgenden Materialien enthalten oder daraus bestehen: Polyamid, Polyester, Polyacryl, Polyurethan, Polypropylen, Polyvinylalkohol, Baumwolle, Viskose und Mischungen daraus. Ein Beispiel für eine mögliche Mischung von Fasern unterschiedlicher Materialien ist eine Kombination von Polyester mit Baumwolle oder von Polyester mit Viskose. Bei Verwendung von Polyamid als Fasermaterial kann das Polyamid in Form von Nylon vorliegen.

Sämtliche der aufgeführten Fasern können zur Verwendung im netzförmigen Substrat als Gewirke ausgestaltet sein. Auch ist es möglich die Fasern als Tüll anzuordnen. Ein Netz in Form eines Tülls kann durch eine repetitive Anordnung von Sechsecken (hexagonale Anordnung innerhalb des Tülls) oder Rauten (Rautenanordnung innerhalb des Tülls) charakterisiert sein.

Alle oben aufgeführten Fasern bzw. Faserarten haben als netzförmiges Substrat vorliegend zum einen strukturgebende Eigenschaften und darüber hinaus die vorteilhafte Eigenschaft Flüssigkeiten wie Wundexsudat an einen optionalen Sekundärverband weiterzuleiten, wo sie gespeichert und später entfernt werden können. Im Allgemeinen sind Fasern natürlichen Ursprungs hydrophiler als synthetische Fasern. Dafür übertreffen synthetische Fasern die Naturfasern hinsichtlich ihrer Bindungsaffinität gegenüber unpolaren Substanzen wie Ölen, Fetten und Wachsen. Zudem sind sie preiswert, reißfest, verfügen über eine hohe Zugfestigkeit und lassen sich einfach verarbeiten. Durch ihren unpolaren Charakter zeigen sie keine Interaktion gegenüber den kationischen und anionischen Verbindungen der antimikrobiellen Inhaltsstoffe. Naturfasern bieten jedoch den Vorteil einer besseren Ökobilanz. Aufgrund ihrer stärkeren Absorption polarer Stoffe, kann es jedoch erforderlich sein, eine größere Menge der Beschichtung aufzutragen, so dass eine ausreichende Beschichtungsmenge an der proximalen Seite außerhalb des Substrates als Wundkontaktfläche zur Verfügung steht.

Daneben ist es auch möglich, dass das netzförmige Substrat nicht aus Fasern, sondern aus einer insbesondere homogenen Masse besteht. Die Masse sollte elastisch, biegsam, beständig und natürlich biokompatibel sein. Eine geeignete Masse ist Silikon, weswegen im Rahmen der Erfindung das netzförmige Substrat aus Silikon bestehen oder solches enthalten kann. Silikon bietet den Vorteil einer größtmöglichen Biokompatibilität. Es ist hypoallergen und weist keinerlei Toxizität auf. Darüber hinaus ist es auch biochemisch innert. Es kann nicht von Mikroorganismen bzw. Krankheitserregern zersetzt oder als Nährsubstrat genutzt werden, verbindet sich nicht mit dem Wundgewebe und kann schmerzfrei abgelöst werden. Aus diesen Gründen wird es gerne im medizinischen Bereich verwendet. Dennoch hat Silikon den Nachteil, dass es aufgrund seiner okklusiven Eigenschaft die Hautatmung behindert und die Absorption von Wundexsudat in Wundauflagen hemmt. Da das Substrat der erfindungsgemäßen Wundkontaktschicht jedoch durch seine netzförmige Struktur über Öffnungen verfügt, bleibt der gewünschte Stoffaustausch auch bei Verwendung von Silikon erhalten.

Zwar ist es im Rahmen der Erfindung grundsätzlich möglich eine Folie als netzförmiges Substrat auszubilden. Bei trockenen oder durch Schorf bedeckten Wunden können folienhaltige netzförmige Substrate eine Alternative zu anderen Substratformen darstellen, wenngleich die Beschichtung bei einer Folie (im Gegensatz zu einem faserbasierten Substrat) nur auf den nach außen weisenden Oberflächen der Folien aufgetragen werden kann. Vorzugsweise ist das von der Wundkontaktschicht umfasste netzförmige Substrat daher keine Folie und enthält auch keine Folie.

Im Rahmen der Erfindung kann das Substrat bei Bedarf frei sein von bestimmten Substanzen. Beispielsweise kann das Substrat frei sein von Gelatine und Kollagen oder generell frei sein von Substanzen tierischen Ursprungs oder von Substanzen natürlichen Ursprungs. Substrate, die frei sind von derartigen Stoffen, sind in aller Regel resistent gegenüber unerwünschtem biologischen Abbau - z.B. beim Behandeln einer infizierten Wunde und hierbei insbesondere bei längerer Anwendung ohne zwischenzeitlichen Verbandswechsel.

Das netzförmige Substrat kann beispielsweise ein Flächengewicht von 50 bis 120 g / m², bevorzugt 70 bis 100 g/m² und besonders bevorzugt 80 bis 90 g/m² aufweisen. Derartige Substrate sind für gewöhnlich so dünn und / oder derart mit Öffnungen versehen, dass der Übergang von Flüssigkeit durch die Wundkontaktschicht in einen möglichen Sekundärverband optimiert ist.

Weiterhin kann die lineare Dichte des netzförmigen Substrats 60 bis 100 dtex, bevorzugt 70 bis 90 dtex gemessen mittels DIN EN ISO 2060 betragen. Die Zähigkeit des Substrats kann 30 bis 50 cN/tex, bevorzugt 35 bis 40 cN/tex gemessen mittels DIN EN ISO 2062 betragen.

Die Wundkontaktschicht kann eine Dehnbarkeit nach DIN EN ISO 1798:2008-04 von mindestens 25%, bevorzugt von mindestens 35% haben. Dehnbarkeit ist dahingehend zu verstehen, dass es bei einer entsprechenden Verlängerung des Materials unter Zugkraft nicht zu einem Belastungsbruch oder zu einem Reißen von Fasern kommt. Bevorzugt ist die Dehnbarkeit eine reversible Dehnbarkeit, so dass das Material nach Aufhebung der Zugkraft im Wesentlichen seine ursprüngliche Länge einnimmt. Eine reversible Dehnbarkeit liegt auch dann vor, wenn das Material nach Aufhebung der Zugkraft eine Länge einnimmt, die maximal 105% der Ausgangslänge entspricht. Die Länge hat dabei dieselbe räumliche Ausrichtung wie die Zugkraft, ist also in Richtung der Zugkraft zu messen. Bevorzugt gelten die angegebenen Dehnungswerte in Faserrichtung. Eine definierte Faserrichtung ist bei den meisten textilen Anordnungen gegeben.

Das netzförmige Substrat kann optional mit elementarem, also nicht ionischem Silber behandelt sein. Dieses zusätzliche Silber, welches in das Substrat eingebettet ist, dient zur Steigerung der antimikrobiellen Wirkung. Da es nicht in der Beschichtung vorhanden ist, sondern von dieser überdeckt wird, wird so eine besonders langfristige antimikrobielle Wirkung (Depoteffekt) erzeugt. Die Fähigkeit von Krankheitserregern sich im Substrat anzusiedeln und zu vermehren ist weiter herabgesetzt oder sogar ganz aufgehoben. Letzteres ist besonders von Vorteil bei infizierten Wunden, die erregerhaltiges Exsudat abgeben, welches von der Wundkontaktschicht aufgenommen oder durch sie hindurchgeleitet wird.

Bedingt durch den netzförmigen Aufbau sind im Substrat Öffnungen enthalten, wobei Abstände zwischen einzelnen Fasern bzw. Faserfilamenten wie sie in jedem Textilverbund (Gewirke, Tüll etc.) vorkommen, nicht als Öffnungen im Sinne der Erfindung gelten.

Die im netzförmigen Substrat vorhandenen Öffnungen können beliebig geformt sein. Möglich ist eine kreisförmige oder elliptische Ausgestaltung, da auf diese Weise Ecken vermieden werden, welche sich nach längerer Tragedauer auf einer Wunde für den Patienten unangenehm anfühlen können. Alternativ ist eine quadratische oder trigonale Öffnungsfläche ebenfalls möglich. Die Größe der Öffnungen kann im Rahmen der vorliegenden Erfindung variieren, wobei diese groß genug ausfallen sollte, um den Abfluss von Exsudat zu ermöglichen bzw. nicht zu verlangsamen. Die Öffnungen können eine Fläche von mindestens 0,01 mm² haben und bevorzugt eine Fläche von mindestens 0,1 mm². Poren sind keine Öffnungen im Sinne der Erfindung. In diesem Zusammenhang können Poren solche Öffnungen sein, die einen Durchmesser von maximal 10 µm oder alternativ maximal 1 µm haben. Besonders gut geeignet sind Öffnungen mit einer Projektionsfläche von mindestens 0,1 mm², entsprechend einem Kreisdurchmesser von 0,357 mm. Andererseits besteht bei zu großen Öffnungen die Gefahr einer Verklebung mit der Wunde und dem Einwachsen von Granulationsgewebe in die Öffnungen. Gut geeignet sind Öffnungen mit einer Größe von 0,1 mm² bis 10 mm², von 0,3 mm² bis 8 mm², besonders 0,5 bis 5 mm². Es ist auch möglich Öffnungen zu nutzen, die eine Öffnungsfläche von maximal 1 mm² haben.

Das Substrat weist eine Vielzahl von Öffnungen und einen geschlossenen Bereich (Stege) auf, der die Öffnungen umgibt. Das Verhältnis der Gesamtfläche der Öffnungen zu der geschlossenen Fläche des Substrats beträgt 1:1,5 bis 1:4, bevorzugt 1:2 bis 1:3.

Bei de im Rahmen der Erfindung eingesetzten Polypeptid oder Polypeptiden kann es sich um Polyarginin, Polylysin und / oder Polyornithin handeln. Somit kann jedes Polypeptidmolekül nur Aminosäuren eines einzigen Typs enthalten. Bei Polylysin kann es sich um α-Poly-L-Lysin und / oder ε-Poly-L-Lysin handeln. Das Polylysin kann beispielsweise eine Molekülmasse von 3,5 bis 4 kDa haben. Weiterhin kann das Polylysin pro Molekül 11 bis 40 Untereinheiten, bevorzugt 15 bis 35 Untereinheiten und besonders bevorzugt 20 bis 50 Untereinheiten Lysin umfassen.

Alle diese genannten Polypeptide tragen aufgrund ihrer chemischen Eigenschaften eine positive Nettoladung und wirken antimikrobiell. Polyarginin hat darüber hinaus zusätzlich den Vorteil die Wundheilung zu fördern, indem der Anteil sogenannter M2 Makrophagen innerhalb der Makrophagen-Population erhöht wird. Während Makrophagen vom Typ M1 Entzündungsreaktionen initiieren und die Produktion von cytotoxischen Radikalen einleiten, wirken M2 Makrophagen entzündungshemmend, proliferativ und fördern den Gewebeverschluss.

Insbesondere wenn von Polypeptiden im Plural die Rede ist kann es sich um folgende Mischungen handeln: a) Polyarginin und Polylysin, b) Polyarginin und Polyornithin, c) Polylysin und Polyornithin sowie eine Mischung aus allen drei dieser Verbindungen. Das Polypeptid bzw. die Polypeptide sind Teil der erfindungsgemäßen Beschichtung. Dabei sind die Anzahlen der Aminosäuren variabel. Demnach können in der Beschichtung Polypeptide mit unterschiedlichen Kettenlängen vorhanden sein.

Weiterhin können Polypeptide Mischungen der drei zuvor genannten Aminosäuren in einem Molekül enthalten. Dabei können die Aminosäuren Polyarginin und Polylysin oder aber Polyarginin und Polyornithin in einem Polypeptid enthalten sein oder das Polypeptid enthält alle drei Aminosäuren.

Bevorzugt enthält die erfindungsgemäße Beschichtung Polyarginin. Der Einsatz derartiger erfindungsgemäßer Polyarginin-haltiger Wundkontaktschichten ist selbst bei nicht infizierten Wunden vorteilhaft, da die Heilung beschleunigt wird.

Darüber hinaus bietet die Kombination aus Polyarginin und Polylysin in der Beschichtung eine besonders ausgeprägte antimikrobielle Wirkung, die vermutlich auf einen synergistischen Effekt zurückzuführen ist und die antimikrobielle Wirkung der jeweiligen Einzelsubstanz übertrifft.

Alternativ kann ein Polypeptid oder Polypeptide mit im Wesentlichen einer einzigen Kettenlänge oder ausschließlich einer einzigen Kettenlänge vorhanden sein. Eine im Wesentlichen einzige Kettelänge liegt dann vor, wenn mindestens 90 %, besser mindestens 95 %, am besten mindestens 98 % und am allerbesten mindestens 99 % aller in der Beschichtung enthaltenen Polypeptide dieselbe Kettenlänge haben. Eine Beschichtung enthaltend Polypeptide derselben oder im Wesentlichen derselben Kettenlänge bieten den Vorteil, dass die antibakterielle Wirkung und die Stabilität der Beschichtung sich sehr gut vorhersagen lassen.

Bevorzugt liegt die Anzahl der Aminosäuren in einem Polypeptid oder in den Polypeptiden bei mindestens 10. Weiterhin liegt die Anzahl der Aminosäuren in einem Polypeptid oder in den Polypeptiden bevorzugt bei höchstens 2000. Somit haben Polypeptid oder Polypeptide im Rahmen der Erfindung eine bevorzugte Kettenlänge von 10 bis 2000 Aminosäuren, besonders bevorzugt 20 bis 1000 Aminosäuren, ganz besonders bevorzugt 25 bis 100 Aminosäuren und am besten 30 bis 50 Aminosäuren. Wie aus den Ausführungsbeispielen hervorgeht, können auch sämtliche Polypeptide in der Beschichtung jeweils 30 Aminosäuren enthalten oder aus 30 Aminosäuren bestehen. Der daraus resultierende Vorteil ist eine besonders starke antimikrobielle Wirkung.

Hyaluronsäure, auch bekannt als Hyaluronan, ist ein zu den Glycosaminoglycanen zählendes Heteropolysaccharid. Grundbaustein der Hyaluronsäure ist ein aus d-Glucuronsäure und N-Acetyl-d-glucosamin alternierend in (1→3)-(1→4)-β-glycosidischer Bindung aufgebautes Aminodisaccharid. Hyaluronsäure ist ein negativ geladenes Polymer, welches auch als Polyanion bezeichnet werden kann. Hyaluronsäure ist wasserbindend und hat gewebsregenerierende und wundheilende Eigenschaften. Eine Möglichkeit Hyaluronsäure zu erhalten, ist diese zu synthetisieren, indem Proteine einer bakteriellen Fermentierung unterzogen werden. Durch eine anschließende Filtrierung wird reine Hyaluronsäure gewonnen. Die für diese Erfindung zu nutzende Hyaluronsäure ist hydrophil. Hyaluronsäure ist Teil der erfindungsgemäßen Beschichtung und trägt aufgrund ihrer chemischen Eigenschaften eine negative Nettoladung.

Hyaluronsäure kann im Rahmen dieser Erfindung in Molmassen von ca. 50 bis ca. 10⁴ kg / mol verwendet werden, bevorzugt wird Hyaluronsäure mit einer molaren Masse von 140 bis 150 kg / mol verwendet. Im Rahmen dieser Erfindung ist es auch möglich, eine Mischung von Hyaluronsäuremolekülen unterschiedlicher Molmassen zu verwenden, wobei in diesem Fall die Molmasse als durchschnittliche Molmasse (Durchschnittsmolmasse) aller Hyaluronsäuremoleküle in der Mischung angegeben werden kann. Beispielsweise kann die durchschnittliche Molmasse 143 bis 146 kg / mol betragen. Alternativ haben alle oder im Wesentlichen alle Hyaluronsäuremoleküle in der Beschichtung dieselbe Molmasse. Eine im Wesentlichen selbe Molmasse liegt vor, wenn mindestens 90 %, besser mindestens 95 %, noch besser mindestens 98% und am besten mindestens 99% der Hyaluronsäuremoleküle in der Beschichtung dieselbe Molmasse haben.

Die erfindungsgemäße Beschichtung enthält mindestens die hierin beschriebene Hyaluronsäure und das hierin beschriebene Polypeptid bzw. die Polypeptide. Weitere Verbindungen oder strukturelle Komponenten können ebenfalls Teil der Beschichtung sein oder mit dieser kombiniert werden. Diese weiteren Verbindungen oder strukturellen Komponenten können flüssig, fest oder gasförmig sein. Darüber hinaus können sie positiv geladen, negativ geladen oder ladungsneutral sein.

In diesem Sinne kann die Beschichtung eine polare Flüssigkeit enthalten. Bei der polaren Flüssigkeit kann es sich um Wasser handeln. Das Wasser kann als destilliertes oder deionisiertes Wasser vorliegen. Bevorzugt liegt die polare Flüssigkeit (z.B. Wasser) als wässrige Pufferlösung vor. Beispiele für wässrige Puffer sind Citratpuffer, Ringerlösung, TRIS-Puffer, TE-Puffer, TBS-Puffer und TBS-T-Puffer. Bevorzugt handelt es sich bei dem Puffer um Tris-NaCl-Puffer. Die Konzentration des Puffers im Lösungsmittel (z. B. Wasser) kann beispielsweise 5 mmol bis 300 mmol betragen, bevorzugt beträgt die Konzentration 10 mmol bis 200 mmol. Im Falle von Tris-NaCl-Puffer kann die Konzentration von Tris beispielsweise 5 bis 300 mmol und die Konzentration von NaCl 10 mmol bis 300 mmol betragen. Alternativ kann die Konzentration des Puffers so gewählt werden, dass die gepufferte Lösung einen pH von 6,8 bis 7,8, bevorzugt 7,2 bis 7,6 hat. Diese pH-Werte beziehen sich zunächst auf die Lösung vor ihrer Vermengung mit anderen Bestandteilen der Beschichtung. Allerdings sind diese Werte auch auf die fertige Beschichtung im finalen Produkt - also der erfindungsgemäßen Wundkontaktschicht - anwendbar, und zwar sowohl vor als auch nach einer optionalen Trocknung.

Die Beschichtung kann einen Konservierungsstoff oder einen Stabilisator in einer Menge von 0,1 bis 2 Gew.-% enthalten. Beispiele für geeignete Konservierungsstoffe sind beispielsweise Benzoesäure, Sorbinsäure oder Parabene. Beispiele für geeignete Stabilisatoren sind Ascorbyl Palmitate und Tocopherol. Da die Wundkontaktschicht mitsamt Beschichtung in der Regel vor der Verwendung sterilisiert wird, kann in den meisten Fällen auf die Verwendung von Konservierungsstoffen verzichtet werden, um den Produktionsaufwand gering zu halten. Vorzugsweise kann auch vorgesehen sein, dass die Beschichtung generell keine Konservierungsstoffe und / oder Stabilisatoren enthält, um die Wahrscheinlichkeit für das Auftreten von Allergien und Unverträglichkeitsreaktionen zu reduzieren. In diesem Sinne kann die erfindungsgemäße Beschichtung und die damit ausgestattete Wundkontaktschicht antiallergen sein.

Der Gehalt an polaren Flüssigkeiten, insbesondere an Wasser, in der Beschichtung kann 1 bis 50 Gew.-% betragen. In manchen Fällen (z.B., wenn die Beschichtung als Gel vorliegen soll) können auch mehr als 50 Gew.-% an polaren Flüssigkeiten gewünscht und sinnvoll sein. Bevorzugt enthält die Beschichtung 15 bis 45 Gew.-% polare Flüssigkeiten, besser 20 bis 40 Gew.-% polare Flüssigkeiten und am besten enthält die Beschichtung 25 bis 35 Gew.-% polare Flüssigkeiten. Diese Konzentrationen können sich auf den finalen Gehalt im fertigen Produkt nach aktiver oder passiver Trocknung beziehen. Es können zwei oder mehr verschiedene polare Flüssigkeiten in der Beschichtung vorhanden sein. Beispiele für mögliche Kombination aus polaren Flüssigkeiten sind Wasser und Ethanol oder Wasser und Glycerin. Darüber hinaus kann es sich bei der polaren Flüssigkeit um einen Puffer wie beispielsweise Tris-NaCl-Puffer handeln. Die polare Flüssigkeit bzw. der Puffer können hierbei einen pH von 6 bis 9, bevorzugt 7 bis 8 und besonders bevorzugt 7,2 bis 7,6 haben.

Die erfindungsgemäße Beschichtung ist eine stabile Beschichtung und bietet eine hervorragende Langzeithaltbarkeit. Diese Langzeitstabilität ist bei medizinischen Artikeln eine besonders gewünschte Eigenschaft, da derartige Produkte von medizinischen Einrichtungen im Rahmen rabattierter Großbestellungen erworben werden und bis zu ihrem Einsatz (der im Regelfall nicht absehbar ist) gelagert werden müssen. Während der Lagerung können die Produkte durchaus saisonal bedingten Temperaturschwankungen unterliegen. Die erfindungsgemäße Beschichtung gewährleistet Beständigkeit sowohl bei verlängertem Lagerungszeitraum als auch gegenüber Temperaturschwankungen. Dies ist auch dem Sterilisationsprozess zuträglich.

Die Beschichtung und somit die beschichtete Wundkontaktschicht hat antimikrobielle, insbesondere antibakterielle Eigenschaften, wobei die antibakteriellen Eigenschaften eine Wirkung gegen die humanpathogenen Keime *S*. *aureus sowie P*. *aeruginosa* umfassen. Die antimikrobielle Wirkung tritt bereits beim Initialkontakt mit den Erregern ein und verstärkt sich im Laufe der Zeit, wobei eine Kontaktzeit von 0 bis 24 Stunden ein bevorzugter Wirkungszeitraum ist.

Gemäß einem Aspekt der Erfindung umfassen das Polypeptid oder die Polypeptide mindestens zehn und / oder höchstens hundert Aminosäuren. Diese Anzahl bezieht sich auf die Menge an Aminosäuren pro Molekül eines Polypeptids. Bevorzugt können das Polypeptid oder die Polypeptide höchsten neunzig, besonders bevorzugt höchstens siebzig, besonders bevorzugt höchstens fünfzig und am besten höchsten vierzig Aminosäuren umfassen. Gleichzeitig oder auch unabhängig von der genannten Obergrenze können das Polypeptid oder die Polypeptide in der Beschichtung mindestens zehn Aminosäuren umfassen. Bevorzugt können das Polypeptid oder die Polypeptide mindesten 20, besonders bevorzugt mindestens 25 und am besten mindestens 28 Aminosäuren umfassen. Die genannten Zahlen beziehen sich auf die Menge an Aminosäuren pro Molekül eines Polypeptids. Typischerweise sind diese Aminosäuren über Peptidbindungen miteinander verbunden. Die genannten Werte können sich auf einen Teil der Polypeptide in der Beschichtung (z.B. mindestens 90 Gew.-%) oder auf sämtliche Polypeptide in der Beschichtung beziehen.

Gemäß einem weiteren Aspekt der Erfindung liegt die Hyaluronsäure in der Beschichtung als Polymer vor, wobei zumindest ein Teil dieser Polymere eine Molekülmasse von mindestens 10 kDa hat. Bevorzugt hat zumindest ein Teil dieser Polymere der Hyaluronsäure eine Molekülmasse von mindestens 15 kDa, besonders bevorzugt von mindestens 20 kDa, ganz besonders bevorzugt von mindestens 25 kDa und am besten von mindestens 30 kDa. Diese Werte können sich alternativ auch auf sämtliche Polymere der Hyaluronsäure in der Beschichtung beziehen.

Im Rahmen der Erfindung kann die Hyaluronsäure als Polymermischung mit unterschiedlicher Kettenlänge vorliegen, wobei zumindest ein Teil der Hyaluronsäurepolymere in der Polymermischung eine Molekülmasse von höchstens 300 kDa hat. Bevorzugt hat zumindest ein Teil dieser Polymere der Hyaluronsäure eine Molekülmasse von höchstens 250 kDa, besonders bevorzugt höchstens 200 kDa, ganz besonders bevorzugt höchstens 150 kDa und am besten höchstens 100 kDa. Dieser Teil kann z.B. 90 Gew.-% der Hyaluronsäure in der Beschichtung ausmachen oder sich alternativ auf sämtliche Hyaluronsäure in der Beschichtung beziehen.

Weiterhin kann die Beschichtung der erfindungsgemäßen Wundkontaktschicht einen Aufbau aus zwei oder mehr Lagen aufweisen. Der Begriff Lage bezieht sich auf eine Schicht innerhalb der Beschichtung. Eine Lage kann in einem Beschichtungsschritt aufgebracht werden. Die erste Lage wird auf dem netzförmigen Substrat der Wundkontaktschicht aufgebracht. Jede weitere Lage - angefangen mit der zweiten Lage - wird auf die zuletzt aufgebrachte Lage aufgebracht. Bevorzugt enthält eine Lage entweder die Hyaluronsäure und hat eine negative Nettoladung oder enthält das Polypeptid bzw. die Polypeptide und hat damit eine positive Nettoladung. Hierbei kann vorgesehen sein, dass eine Lage, die die Hyaluronsäure enthält, frei ist von dem Polypeptid bzw. den Polypeptiden und eine Lage, die das Polypeptid bzw. die Polypeptide enthält, frei ist von der Hyaluronsäure.

In diesem Sinne kann die Beschichtung mindestens zwei übereinanderliegende und miteinander verbundenen Lagen enthalten, wobei mindestens eine Lage vorhanden ist, welche das Polypeptid oder die Polypeptide enthält und eine positive Nettoladung hat sowie mindestens eine Lage, welche die Hyaluronsäure enthält und eine negative Nettoladung hat, und wobei sich bei den übereinanderliegenden Lagen jeweils eine das Polypeptid oder die Polypeptide enthaltende Lage mit einer die Hyaluronsäure enthaltende Lage abwechselt, so dass eine Abfolge von alternierenden Lagen gebildet wird.

Die Anzahl der alternierenden Lagen kann dabei eine gerade oder ungerade Zahl sein. Eine gerade Anzahl an alternierenden Lagen ist dabei bevorzugt, weil dabei für jede negativ geladene Lage eine positiv geladene Lage zur Verfügung steht und sich die gegensätzlichen Ladungen dabei anziehen, was zu einer besonders stabilen Beschichtung führt.

Vorzugsweise ist die Anzahl der alternierenden Lagen 20 bis 100, bevorzugt 30 bis 90, besonders bevorzugt 40 bis 80 und ganz besonders bevorzugt 50 bis 70. Dabei enthält jeweils die Hälfte der Lagen die Hyaluronsäure und die andere Hälfte der Lagen das Polypeptid oder die Polypeptide. Bei einer ungeraden Anzahl an alternierenden Lagen überwiegt bevorzugt die Anzahl derjenigen Lagen, die das Polypeptid enthalten.

Im Rahmen der Erfindung können die Hyaluronsäure und das Polypeptid bzw. die Polypeptide innerhalb der Beschichtung jedoch auch durchmischt vorliegen. Durchmischt ist in diesem Sinne als Gemisch im chemischen Sinne zu verstehen. Das Gemisch ist hierbei bevorzugt eine Lösung, und zwar eine Lösung mit nur einer Phase. Bevorzugt sind die Hyaluronsäure und das Polypeptid bzw. die Polypeptide innerhalb der Beschichtung homogen verteilt. Weiterhin bevorzugt ist das Polypeptid oder die Polypeptide in einer Hyaluronsäurematrix eingebettet. Das Hyaluronsäuregel besteht aus Hyaluronsäure und Wasser. Die Hyaluronsäurematrix bildet sich aus, wenn die Beschichtung im trockenen Zustand vorliegt.

Eine durchmischte Beschichtung im Sinne des eben genannten lässt sich durch Aufsprühen einer Lösung enthaltend Hyaluronsäure und Aufsprühen einer Lösung enthaltend das Polypeptid bzw. die Polypeptide auf das netzförmige Substrat erhalten. Die beiden Lösungen können nacheinander oder gleichzeitig gesprüht werden. Sobald eine Lage erhalten wurde und diese getrocknet oder angetrocknet ist, können weitere Lagen aufgesprüht oder mittels Tauchverfahren hinzugefügt werden.

Eine durchmischte Beschichtung kann eine oder mehrere Lagen enthalten, von denen mindestens eine Lage sowohl die Hyaluronsäure als auch das Polypeptid bzw. die Polypeptide enthält. In diesem Sinne ist eine solche durchmischte Beschichtung gänzlich oder teilweise von der Mischung durchdrungen.

Eine Möglichkeit die Lagen der Beschichtung aufzutragen, besteht darin, diese mittels Tauchverfahren aufzubringen. Das Tauchverfahren ist darüber hinaus das geeignete Verfahren, um solche Lagen innerhalb der Beschichtung zu erzeugen, die jeweils entweder Hyaluronsäure ohne das Polypeptid bzw. die Polypeptide oder das Polypeptid bzw. die Polypeptide ohne die Hyaluronsäure enthalten. So ist es beim Tauchverfahren möglich zwei Lösungen in zwei separaten Kompartimenten bereitzustellen, wobei ein Kompartiment die Lösung mit der Hyaluronsäure und das andere Kompartiment die Lösung mit dem Polypeptid bzw. den Polypeptiden enthält. Auf das netzförmige Substrat können auf diese Weise durch abwechselndes Eintauchen in den beiden Kompartimenten Lagen aufgetragen werden, bis die Beschichtung vollständig ist.

Die Anzahl der übereinanderliegenden und miteinander verbundenen Lagen in der Beschichtung kann beispielsweise 10 bis 100 betragen. Bevorzugt beträgt die Anzahl der übereinanderliegenden und miteinander verbundenen Lagen 15 bis 90, besonders bevorzugt 20 bis 80, ganz besonders bevorzugt 25 bis 70 und am besten 30 bis 60.

Gemäß einem Konzept der Erfindung haben das Polypeptid bzw. die Polypeptide eine Molekülmasse von 1 bis 41 kDa. Bevorzugt haben das Polypeptid bzw. die Polypeptide eine Molekülmasse von 2 bis 40 kDa, besonders bevorzugt 3 bis 39 kDa, ganz besonders bevorzugt 4 bis 38 kDa und am besten 5 bis 37 kDa. Möglich ist auch, dass mindestens eine, mindestens zwei oder mindestens drei Lagen der Beschichtung ein Polypeptid bzw. Polypeptide mit einer solchen Molekülmasse hat.

Die Beschichtung der erfindungsgemäßen Wundkontaktschicht kann eine Dicke von 10 nm bis 1000 nm haben. Bevorzugt hat die Beschichtung eine Dicke von 50 nm bis 900 nm, besonders bevorzugt eine Dicke von 100 nm bis 800 nm, besonders bevorzugt eine Dicke von 150 nm bis 700 nm und am besten 200 nm bis 600 nm. Die Dicke kann dabei gemessen werden von der Oberkante der Grundfläche des netzförmigen Substrats, und zwar ohne, dass das Substrat während der Messung gestaucht oder komprimiert wird. Empfohlen wird die Dicke der Beschichtung durch die Anzahl der Lagen einzustellen. Die Dicke nimmt mit zunehmender Anzahl der Lagen weiter zu. Beschichtungen, deren Dicke eine größere Ausdehnung erreicht, haben besonders ausgeprägte atraumatische Eigenschaften. Beschichtungen, die eine geringe Dicke aufweisen, ermöglichen einen schnelleren Durchfluss von Körperflüssigkeiten aus der Wunde durch die erfindungsgemäße Wundkontaktschicht in einen möglichen Sekundärverband.

Weiterhin ist die erfindungsgemäße Beschichtung der Wundkontaktschicht vorzugsweise trocknungsbeständig. In diesem Sinne können die Bestandteile der Beschichtung - wie Hyaluronsäure und Polypeptid oder Polypeptide - in feuchtem Zustand als Lösung aufgetragen werden, um anschließend zu trocknen. Das Trocknen kann passiv bei Raumtemperatur erfolgen oder aktiv unter Einsatz technischer Hilfsmittel, wobei letzteres im Allgemeinen deutlich schneller, aber auch energieaufwändiger ist. Die Beschichtung behält auch im getrockneten Zustand ihre Eigenschaften - insbesondere die atraumatische und antimikrobielle Wirkung - bei. Die Trocknungsbeständigkeit der Beschichtung hat den Vorteil, dass sich trockene Wundversorgungsprodukte einfacher im industriellen Maßstab verarbeiten und verpacken lassen. Darüber hinaus lassen sich trocknungsbeständige Wundversorgungsprodukte leichter und länger lagern, da sie nicht gegen ein ungewolltes Eintrocknen geschützt werden müssen.

Die Beschichtung gilt beispielsweise als trocken, wenn der Flüssigkeitsgehalt (z.B. Wassergehalt) in der Beschichtung maximal 5 Gew.-%, bevorzugt maximal 4 Gew.-%, besonders bevorzugt maximal 3 Gew.-% und am besten maximal 1 Gew.-% beträgt. Geringe Abweichungen von diesen Werten können sich in Abhängigkeit von der Konzentration gelöster Teilchen, deren Hydrophilie und Molmasse ergeben und sind von der technischen Lehre der Erfindung eingeschlossen.

Von der Erfindung ebenfalls umfasst ist die hierin beschriebene Wundkontaktschicht, wobei das netzförmige Substrat mindestens eine der folgenden Fasern enthält oder daraus besteht: Polyamidfasern, insbesondere Nylonfasern, Polyesterfasern, Baumwollfasern, Viskosefasern. Synthetische Fasern wie Polyamid und Polyester haben den Vorteil, dass sie beständig sind gegen Zersetzung durch Mikroorganismen.

Weiterhin kann das netzförmige Substrat ein Vlies, Gewirke oder Tüllgewebe sein.

Insbesondere kann das Vlies, Gewirke oder Tüllgewebe aus den oben genannten Faserarten bestehen oder diese enthalten. Bevorzugt sind in diesem Fall Polyester und / oder Polyamid.

Ferner von der Erfindung umfasst sind Verfahren zur Herstellung der hierin beschriebenen Wundkontaktschicht und zum Auftragen der Beschichtung auf das netzförmige Substrat.

Nachfolgend wird ein Verfahren beschrieben, mit dem man eine Wundkontaktschicht mit durchmischten Beschichtungen und / oder mit durchmischten Lagen innerhalb der Beschichtung erzeugen kann:
i) Bereitstellung eines netzförmigen Substrates
ii) Besprühen des netzförmigen Substrates mit Hyaluronsäure und einem Polypeptid oder Polypeptiden zur Ausbildung einer Beschichtung umfassend mindestens eine Lage,
wobei es sich bei dem Polypeptid oder den Polypeptiden um Polyarginin und / oder Polylysin handelt und wobei die mindestens eine Lage sowohl die Hyaluronsäure als auch das Polypeptid enthält.

Das Besprühen kann beispielsweise mittels eines Zerstäubers oder einer Sprühpistole geschehen. Bevorzugt handelt es sich um ein elektrisches Sprühgerät, das eine Konstante Menge an Flüssigkeitsvolumen pro Zeiteinheit versprüht.

Einer der großen Vorteile dieses Verfahrens ist die Zeitersparnis. In diesem Sinne kann vorgesehen sein, dass das Aufsprühen einer Lage maximal 5 min, bevorzugt maximal 1 min, besonders bevorzugt maximal 30 s und ganz besonders bevorzugt maximal 5 s dauert.

Weiterhin kann vorgesehen sein, dass der gesamte Beschichtungsprozess mittels Aufsprühen vom Beginn des Sprühvorgangs bis zum Erhalt der fertigen Wundkontaktschicht maximal 30 min dauert, wobei die Trocknung der Wundkontaktschicht in diesem Zeitraum bereits inkludiert sein kann.

Bevorzugt liegen die Hyaluronsäure und das Polypeptid oder die Polypeptide während des Besprühens gemäß Schritt ii) in Lösung vor. Mögliche Lösungen wie polare Flüssigkeiten, insbesondere Wasser und wässrige Pufferlösungen wurden oben bereits beschrieben und können im Rahmen des zuletzt genannten Verfahrens eingesetzt werden.

Bevorzugt beträgt die Konzentration des in Lösung vorliegenden Polypeptids oder der Polypeptide während des Besprühens gemäß Schritt ii) bei 0,1 mg / ml bis 100 mg / ml, besonders bevorzugt bei 1 mg / ml bis 80 mg / ml, ganz besonders bevorzugt bei 3 mg / ml bis 50 mg / ml und am besten 5 mg / ml bis 30 mg / ml.

Bevorzugt beträgt die Konzentration der Hyaluronsäure während des Besprühens gemäß Schritt ii) bei 0,1 mg / ml bis 10 mg / ml, besonders bevorzugt 0,5 mg / ml bis 8 mg /ml, ganz besonders bevorzugt bei 1 mg / ml bis 5 mg / ml und am besten 2 mg / ml bis 4 mg / ml.

Bevorzugt werden durch das Besprühen gemäß Schritt ii) 0,1 bis 1 ml einer solchen Lösung des Polypeptids bzw. der Polypeptide und / oder 0,1 ml bis 1 ml einer solchen Lösung der Hyaluronsäure pro cm² der proximalen (wundzugewandten) Seite des netzförmigen Substrats aufgesprüht. Besonders bevorzugt werden 0,2 bis 0,9 ml, ganz besonders bevorzugt 0,3 bis 0,8 ml und am besten 0,4 bis 0,7 ml einer solchen Lösung aufgesprüht. Aussparungen und Öffnungen die durch die netzförmige Ausgestaltung des Substrats auftreten, sind bei der Flächenbestimmung unberücksichtigt zu lassen. Bei einem Substrat mit den Außenmaßen 10 cm x 10 cm soll also davon ausgegangen werden, dass das Substrat eine Fläche von 100 cm2 hat. Die Volumenangaben sind pro Lage zu verstehen. Da im Falle durchmischter Lagen bereits eine einzige Lage eine fertige Beschichtung ausbilden kann, können die Volumenangaben in diesem Sinne auch pro Beschichtung (enthaltend eine Lage) verstanden werden.

Gemäß einem weiteren Aspekt der Erfindung wird bei dem oben beschriebenen Verfahren das Besprühen gemäß Schritt ii) mindestens zwei Mal ausgeführt, um eine Beschichtung mit mindestens zwei Lagen zu erzeugen. Zwischen dem Besprühen kann eine Trocknungsphase liegen. Diese kann 30 s bis 30 min betragen, bevorzugt 1 min bis 25 min, ganz besonders bevorzugt 2 min bis 20 min und am besten 3 min bis 15 min.

Teil der Erfindung ist auch eine Wundkontaktschicht wie hierin beschrieben, erhältlich oder erhalten durch das zuletzt erläuterte Herstellungsverfahren.

Ein anderes erfindungsgemäßes Verfahren zur Herstellung einer Wundkontaktschicht umfasst die folgenden Schritte:
i) Bereitstellung eines netzförmigen Substrates
ii) Beschichten einer Oberfläche des netzförmigen Substrates, durch Auftragen von mindestens zwei übereinanderliegenden Lagen, wobei mindestens eine Lage ein Polypeptid oder Polypeptide enthält, wobei es sich um Polyarginin und / oder Polylysin handelt und eine positive Nettoladung hat und mindestens eine Lage Hyaluronsäure enthält und eine negative Nettoladung hat und wobei die übereinanderliegenden Lagen in ihrer Nettoladung alternieren. Eine alternierende Anordnung der Lagen ist oben näher definiert.

Der Beschichtungsschritt ii) kann insbesondere mittels Tauchverfahren ausgeführt werden. Andere Beschichtungstechniken sind ebenfalls möglich. So kann das Beschichten beispielsweise alternativ auch durch Auftragung mittels Walze oder durch Bestreichen (z.B. mittels Pinsel) erfolgen.

Empfohlen wird sowohl die Hyaluronsäure als auch das Polypeptid oder die Polypeptide vor dem Beschichten in Lösung zu bringen. Geeignete Lösungsmittel und Konzentrationen wurden hierin bereits an anderer Stelle beschrieben und sind im Rahmen des zuletzt genannten Verfahrens anwendbar. So können das Polypeptid oder die Polypeptide beispielsweise mittels einer Lösung aufgebracht werden, in der sie in einer Konzentration von 0,1 mg / mL bis 100 mg / mL enthalten sind und / oder beispielsweise die Hyaluronsäure mittels einer Lösung aufgetragen werden, die die Hyaluronsäure in einer Konzentration von 0,1 bis 10 mg / mL enthält.

Dieses Verfahren eignet sich insbesondere zur Herstellung solcher Wundkontaktschichten, deren Beschichtung mindestens zwei nicht durchmischte Lagen enthält. In der Regel enthalten die mit dem obigen Verfahren erzeugten Beschichtungen mindestens zwei Lagen. Bevorzugt sind sämtliche Lagen der mittels dieses Verfahrens erzeugten Beschichtung nicht durchmischt.

Im Rahmen des zuletzt genannten Verfahrens ist es möglich eine Vielzahl von Lagen zu einer Beschichtung zu vereinen. Empfohlen wird im Rahmen von Schritt ii) nach dem Auftragen einer Lage diese mit einer Pufferlösung zu spülen. Dies sollte erst dann geschehen, wenn die zu spülende Lage ausreichend getrocknet oder angetrocknet ist, um ein ungewolltes Abwaschen zu vermeiden.

Geeignete Pufferlösungen und pH-Werte sind hierin bereits an anderer Stelle genannt worden. Bevorzugt wird hierzu wässrige Pufferlösung von Tris-NaCl verwendet. Besonders bevorzugt sind sowohl Tris als auch NaCL in einer Konzentration von 10 mmol bis 150 mmol in der Pufferlösung vorhanden. Die Konzentrationen von Tris und NaCl müssen dabei nicht identisch sein.

Weiterhin kann das Beschichten gemäß Schritt ii) des zuletzt genannten Verfahrens durch (vollständiges oder teilweises) Eintauchen des netzförmigen Substrates in eine Lösung, die das Polypeptid oder die Polypeptide enthält und / oder in eine Lösung, die die Hyaluronsäure enthält, erfolgen.

Das (vollständige oder teilweise) Eintauchen kann dabei für die Erzeugung einer einzelnen Lage über eine Zeitspanne von ein bis zehn Minuten erfolgen. Das heißt, dass das netzförmige Substrat in die Lösung eingetaucht wird und nach ein bis zehn Minuten entnommen wird. Die folgenden alternativen Zeitspannen sind ebenfalls möglich: zwei bis neun Minuten, drei bis acht Minuten sowie vier bis sieben Minuten.

Teil des zuletzt genannten Verfahrens ist darüber hinaus, dass Schritt ii) wiederholt stattfinden kann (also mindestens zwei Mal). So kann Schritt ii) für die Herstellung einer erfindungsgemäßen Wundkontaktschicht beispielsweise zehn bis hundert Mal wiederholt werden, so dass zehn bis hundert Lagen übereinanderliegend aufgetragen werden. Dies resultiert folglich in einer Wundkontaktschicht, deren netzförmiges Substrat eine Beschichtung aufgetragen ist, die auch zehn bis hundert Lagen besteht.

Andere Wiederholungsanzahlen für Schritt ii) sind 15 bis 90 Mal, 20 bis 80 Mal, 25 bis 70 Mal und 30 bis 60 Mal.

Wenn die Beschichtungsauftragung gemäß Schritt ii) mittels Tauchverfahren ausgeführt wird, beziehen sich die eben genannten Wiederholungszahlen auf die Anzahl des Eintauchens in einer Lösung enthaltend die Hyaluronsäure oder das Polypeptid bzw. die Polypeptide.

Teil der Erfindung ist auch eine Wundkontaktschicht wie hierin beschrieben, erhältlich oder erhalten durch das zuletzt erläuterte Herstellungsverfahren.

Die hierin beschriebenen Herstellungsverfahren können sich bei Bedarf durch folgende Punkte auszeichnen:
- Beide Seiten des netzförmigen Substrats (proximale und distale Oberfläche) können gleichzeitig beschichtet werden.
- Auf ein netzförmiges Substrat mit negativer Nettoladung oder negativer Außenladung (negative Ladung der Oberfläche) kann zuerst eine Lage enthaltend das Polypeptid oder die Polypeptide als erste Lage aufgetragen werden. In diesem Fall hat die erste Lage eine positive Nettoladung. Bevorzugt enthält in diesem Fall die erste Ladung keine Hyaluronsäure.
- Auf ein netzförmiges Substrat mit positiver Nettoladung oder positiver Außenladung (positive Ladung der Oberfläche) kann zunächst eine Lage enthaltend die Hyaluronsäure als erste Lage aufgetragen werden. In diesem Fall hat die erste Lage eine negative Nettoladung. Bevorzugt enthält in diesem Fall die erste Ladung kein Polypeptid bzw. keine Polypeptide.

Darüber hinaus umfasst die Erfindung auch ein Kit umfassend die erfindungsgemäße Wundkontaktschicht und weiterhin enthaltend einen Sekundärverband oder ein Befestigungsmittel, wobei der Sekundärverband oder das Befestigungsmittel geeignet sind zur Befestigung der Wundkontaktschicht an einer Wunde. Dabei kann auch vorgesehen sein, dass die Wunde durch die Wundkontaktschicht abgedeckt wird.

Ein weiterer Teil der Erfindung betrifft die hierin beschriebenen Herstellungsverfahren, wobei das bereitgestellte netzförmige Substrat zunächst einer Plasmareinigung unterzogen wird. Die Plasmareinigung findet vor der Beschichtung, Auftragung oder dem Besprühen statt und erlaubt ein noch besseres Anhaften der späteren Beschichtung auf dem Substrat.

Weiterhin können die hierin beschriebenen Herstellungsverfahren automatisiert sein. Bevorzugt findet die Automatisierung mittels eines programmierbaren Roboters statt. Insbesondere das Auftragen der Beschichtung mittels Tauchverfahren profitiert von einer Automatisierung, da diese Methode in der Regel mehr Zeit beansprucht als das Auftragen der Beschichtung mittels Sprühverfahren. Bevorzugt werden bei dem automatisierten Tauchverfahren Beschichtungen mit mindestens 20, besser 25 und am besten 30 Lagen auf dem netzförmigen Substrat erzeugt.

Das Sprühverfahren profitiert ebenfalls von einer Automatisierung, da hierdurch ein gleichbleibender Abstand zwischen Sprühdüse und Substrat während des Sprühvorgangs gewährleistet wird, was bei einem händischen Ansatz deutlich schwieriger umzusetzen ist.

Die erfindungsgemäße Wundkontaktschicht kann auf dem netzförmigen Substrat eine Beschichtung mit einem Flächengewicht von beispielsweise 5 ng bis 200 ng / cm² Substrat enthalten. Bevorzugt hat die Beschichtung ein Flächengewicht von 10 ng bis 150 ng / cm² Substrat, besonders bevorzugt ein Flächengewicht von 15 ng bis 130 ng / cm² Substrat, ganz besonders bevorzugt ein Flächengewicht von 20 ng bis 100 ng / cm² Substrat und am besten 20 ng bis 50 ng / cm² Substrat. Das Flächengewicht der Beschichtung bezieht sich dabei auf die Summe der Massen aus Polypeptid bzw. Polypeptiden und Hyaluronsäure.

Das Flächengewicht des Polypeptids bzw. der Polypeptide in der Beschichtung kann beispielsweise bei 1 ng bis 500 ng / cm² Substrat, bevorzugt 2 ng bis 300 ng / cm² Substrat und ganz besonders bevorzugt 3 ng bis 50 ng / cm² Substrat betragen.

Das Flächengewicht der Hyaluronsäure in der Beschichtung kann beispielsweise bei 3 ng bis 1.000 mg / cm² Substrat, bevorzugt 6 ng bis 400 ng / cm² Substrat und besonders bevorzugt 7 ng bis 50 ng / cm² Substrat betragen.

Das Verhältnis von Polypeptid bzw. Polypeptiden zu Hyaluronsäure in der Beschichtung kann beispielsweise ein Massenverhältnis von 0,5 zu 1 bis 5 zu 1 haben. Bevorzugt liegt das Verhältnis bei 1 zu 1 bis 4 zu 1, besonders bevorzugt liegt das Verhältnis bei 1, 5 zu 1 bis 3,5 zu 1 und ganz besonders bevorzugt bei 2 zu 1 bis 3,5 zu 1.

Die Masse der Puffersubstanzen in Form einer Base oder einer Säure sowie einem geeigneten Salz dieser Base oder Säure kann ein Flächengewicht in der Beschichtung von beispielsweise 50 ng bis 1.000 ng / cm² Substrat ausmachen. Bevorzugt haben die Puffersubstanzen in der Beschichtung ein Flächengewicht von 100 ng bis 500 ng / cm² Substrat, besonders bevorzugt ein Flächengewicht von 120 ng bis 300 ng / cm² Substrat. Bevorzugt enthält die Puffersubstanz eine Base, besonders bevorzugt handelt es sich um Tris und ganz besonders bevorzugt wird die Base Tris kombiniert mit dem Salz NaCl.

Die Wundkontaktschicht kann die antimikrobielle Beschichtung in folgenden Varianten enthalten, wobei diese Aufzählung exemplarisch und nicht abschließend zu verstehen ist:
a) Beschichtung umfassend mindestens zwei Lagen, von denen mindestens eine Lage Hyaluronsäure, aber kein Polypeptid enthält und von denen mindestens eine Lage ein Polypeptid oder Polypeptide aber keine Hyaluronsäure enthält. Falls mehr als zwei Lagen vorhanden sind, sind diese alternierend angeordnet.
b) Durchmischte Beschichtung umfassend mindestens eine Lage, die sowohl Hyaluronsäure als auch das Polypeptid bzw. die Polypeptide enthält.
c) Eine Kombination aus a) und b).

In diesem Sinne umfasst die Erfindung eine Wundkontaktschicht, die beidseitig beschichtet ist und bei der beide Seiten unabhängig voneinander mit einer Beschichtung gemäß a), b) oder c) ausgestattet sind.

Sofern die Wundkontaktschicht ein netzförmiges Substrat umfasst, dessen erste und zweite Seite mit der Beschichtung beschichtet ist, bietet das den Vorteil, dass der Anwender nicht entscheiden muss, welche Seite zum Auflegen auf die Wunde konfiguriert ist, was die Wahrscheinlichkeit von Fehlern reduziert und die Arbeit im klinischen Alltag erleichtert.

Zusätzlich können die Inhaltsstoffe der antimikrobiellen Beschichtung in einem bestimmten Mengenverhältnis zueinanderstehen. So kann das Gewichtsverhältnis der Gewichtskonzentration von Hyaluronsäure zur Summe der Gewichtskonzentrationen der des Polypeptids bzw. der Polypeptide in der Beschichtung von 9 zu 1 bis hin zu 1 zu 80 reichen, bevorzugt 4 zu 1 bis hin zu 1 zu 20.

Ferner kann das Stoffmengenverhältnis der Stoffmenge von Hyaluronsäure zur Summe der Stoffmenge des Polypeptids oder der Stoffmengen der Polypeptide von 4 zu 25 bis hin zu 1 zu 500 reichen. Die Stoffmengenbestimmung kann in der Standardeinheit mol vorgenommen werden.

Das netzförmige Substrat kann teilweise oder vollständig mit der Beschichtung beschichtet sein. Insbesondere können mindestens 80 % der Fläche der ersten Seite des Substrats beschichtet sein. Bevorzugt sind 90 % der Fläche der ersten Seite des Substrats beschichtet. Besonders empfohlen wird mindestens 99 % dieser Fläche zu beschichten. Hierbei werden Öffnungen im Substrat (wie bereits weiter oben ausgeführt) bei der Flächenbestimmung nicht berücksichtigt. In diesem Zusammenhang sei darauf hingewiesen, dass die Beschichtung ein stabiler Teil der Wundkontaktschicht ist, aber für Wasser bzw. wässrige Wundflüssigkeiten passierbar ist. Auf diese Weise kann überschüssiges Wundexsudat in einen oberhalb der Wundkontaktschicht angebrachten Sekundärverband aufgenommen und dort gespeichert werden. Der Sekundärverband kann später unabhängig von der Wundkontaktschicht gewechselt und das Exsudat dabei entsorgt werden.

Die resultierende Beschichtungsmenge kann durch Wiegen des behandelten Substrats ermittelt werden. Beschichtungen mit größerem Flächengewicht können durch wiederholte Einzelbeschichtungen erzeugt werden.

Das netzförmige Substrat der Wundkontaktschicht kann mindestens eines der folgenden Materialien enthalten oder daraus bestehen: Polyamid, insbesondere Nylon, Polyester, Baumwolle, Viskose oder eine Kombination aus zwei oder mehr der aufgeführten Materialien. Die besagten Materialien können in Form von Fasern vorliegen. Beispielsweise kann es sich um Polyamidfasern, insbesondere Nylonfasern, Polyesterfasern, Baumwollfasern oder Viskosefasern handeln. Das Substrat kann dabei in Form eines Polyamidgewirkes vorliegen, insbesondere kann es als Nylongewirke vorliegen. Darüber hinaus können auch Mischfasern verwendet werden, welche eine Kombination aus zwei oder mehr der aufgeführten Materialien in sich vereinen.

Sowohl die erste und optional auch die zweite Seite des netzförmigen Substrats kann mit einer Beschichtung mit dem oben genannten Flächengewicht ausgestattet sein. Es hat sich gezeigt, dass diese Menge zu hervorragenden Ergebnissen bei der Förderung der Wundheilung und der Bekämpfung von Krankheitserregern in der Wunde führt. Gleichzeitig ist es möglich, ein stabiles Produkt mit ausreichend langer Lagerfähigkeit zu erhalten, und zwar ohne, dass sich die Beschichtung vom Substrat löst. Bevorzugt handelt es sich bei der Beschichtung um eine gleichmäßige oder im Wesentlichen gleichmäßige Verteilung der Beschichtungsmasse, bei der jeder Bereich der präparierten Seite der Wundkontaktschicht mit derselben oder im Wesentlichen derselben Menge an Beschichtung bestückt ist.

Die erfindungsgemäße Beschichtung der Wundkontaktschicht ist sterilisationsbeständig und behält ihre funktionellen und strukturellen Eigenschaften nach der Sterilisation bei. Insbesondere bleiben die antimikrobiellen und atraumatischen Eigenschaften erhalten. Weiterhin bleiben die strukturellen Eigenschaften erhalten, so dass sich die Beschichtung nicht löst oder zerfließt. Die Sterilisation kann dabei beispielsweise mittels Ethylenoxid, Dampfsterilisation (Autoklavierung) oder Heißluftsterilisation erfolgen. Bevorzugt ist die gesamte Wundkontaktschicht (also inklusive Beschichtung und netzförmigem Substrat) sterilisationsbeständig. Dies kann beispielsweise dadurch erreicht werden, dass das Substrat aus solchen Fasern oder Materialien aufgebaut ist, wie sie an anderer Stelle hierin beschrieben sind.

Die Sterilisationsbeständigkeit ist eine wichtige Eigenschaft von Wundversorgungsprodukten, da von Materialien, die in direktem Kontakt mit der Wunde kommen, in der Regel Keimfreiheit gefordert wird.

Die Wundkontaktschicht kann neben dem netzförmigen Substrat und der erfindungsgemäßen antimikrobiellen Beschichtung weitere Verbindungen oder Strukturelemente enthalten. Hierzu gehören insbesondere Substanzen, die die Heilung fördern, die Wundränder vor Mazeration schützen und/oder die antimikrobielle Wirkung verbessern. Diese Substanzen können in die Beschichtung eingearbeitet, auf die proximale Seite der Beschichtung aufgetragen oder Teil des netzförmigen Substrates sein. Beispiele für Substanzen mit wundheilungsfördernder Wirkung sind Allantoin oder Dexpanthenol. Ein Beispiel für Substanzen mit antimikrobieller Wirkung sind Silber (kationisch oder elementar) und PHMB (Polyhexamethylenbiguanid).

Die Erfindung betrifft darüber hinaus eine Wundauflage, die die erfindungsgemäße Wundkontaktschicht umfasst. Die Wundkontaktschicht liegt dabei als eine Außenschicht der Wundauflage vor, so dass deren Beschichtung während der Anwendung in Kontakt mit der Haut bzw. der Wunde steht (proximale Ausrichtung der Wundkontaktschicht innerhalb der Wundauflage). Vor der Verwendung kann die erste Seite der Wundkontaktschicht durch eine Schutzschicht wie beispielsweise einen Release-Liner überdeckt sein. Die Schutzschicht wird vor der Verwendung entfernt.

Bevorzugt umfasst diese Wundauflage eine Absorptionsschicht, welche auf der zweiten Seite der Wundkontaktschicht angebracht ist und / oder einen umlaufenden Kleberand zum Ankleben der Wundauflage an die Haut, welche die Wunde umgibt und / oder eine rückseitige (oberhalb der zweiten Seite der Wundkontaktschicht gelegene) Stützschicht (Backing). Die Absorptionsschicht kann Vliesgewebe, Superabsorber, Flockenzellstoff oder ein geschäumtes Material umfassen. Die Stützschicht kann als Folie ausgebildet sein, die optional durchlässig für Wasserdampf, aber nicht für flüssiges Wasser ist und die ein Polyurethan (PU) enthalten oder daraus bestehen kann. Der umlaufende Kleberand kann Teil der Stützschicht sein. Eine Absorptionsschicht bietet sich vor allem für blutende oder stark exsudierende Wunden an. Eine Stützschicht hat den Vorteil, dass Wunde und Wundkontaktschicht vor Nässe von außen geschützt werden.

Ein weiterer Aspekt der Erfindung betrifft die erfindungsgemäße Wundkontaktschicht oder eine Wundauflage, die diese Wundkontaktschicht umfasst, zur Anwendung in einem Verfahren zur Behandlung von Wunden, bevorzugt von infizierten Wunden, besonders bevorzugt von Wunden, die mit *S*. *aureus* und / oder *P*. *aeruginosa* infiziert sind.

Ein weiterer Aspekt der Erfindung betrifft die antimikrobielle Beschichtung zur Anwendung in einem Verfahren zur Behandlung von Wunden, bevorzugt von infizierten Wunden, wobei die Beschichtung auf einem netzförmigen Substrat, welches als Wundkontaktschicht vorgesehen ist, vorliegt.

Bei diesen beiden Anwendungsfällen kann vorgesehen sein, dass die Anwendung über einen Zeitraum von 0 bis 24 Stunden stattfindet oder, dass die Anwendung in einem

Zeitraum von mindestens 24 Stunden stattfindet, wobei letzteres beispielswiese bei bereits stark ausgeprägten Infektionen oder bei Patienten mit Immunschwäche geboten sein kann.

Die erfindungsgemäße Wundkontaktschicht ist geeignet zur Abdeckung und / oder Therapie von Wunden, insbesondere infizierten Wunden. Aus diesen Gründen kann die Wundauflage weiterhin eingesetzt werden in einem Verfahren zur Wundtherapie und / oder zur Reduktion der Keimzahl in Wunden.

Eine besonders bevorzugte Ausführungsform betrifft eine erfindungsgemäße Wundkontaktschicht wobei das netzförmige Substrat ein Tüllgewebe ist, das Polyester oder Polyamid enthält und wobei die Beschichtung 48 alternierende Lagen enthält, wovon 24 Lagen Hyaluronsäure enthalten und 24 Lagen das Polypeptid enthalten, welches entweder Polylysin ist oder Polyarginin ist, wobei im Falle von Polyarginin jedes Molekül 30 Aminosäuren vom Typ Arginin umfasst und im Falle von Polylysin jedes Molekül 20 bis 40 Aminosäuren vom Typ Lysin umfasst.

Daneben betrifft die Erfindung auch die Verwendung der Wundkontaktschicht zur Herstellung der oben beschriebenen Wundauflage oder die Verwendung der beschriebenen antimikrobiellen Beschichtung in einem Verfahren zur Herstellung der Wundkontaktschicht.

Ein weiterer Aspekt der Erfindung ist ein Kit enthaltend die erfindungsgemäße Wundkontaktschicht in Kombination mit einem Sekundärverband. Der Sekundärverband sollte in der Lage sein die Wundkontaktschicht an der Wunde zu fixieren. Dazu kann der Sekundärverband beispielsweise über Klebemittel verfügen oder über die Wundkontaktschicht und den betreffenden Körperbereich gewickelt werden.

Bei dem Kit kann es sich um eine Verpackung oder ein Set handeln.

### Figuren

Im Folgenden werden die Figuren näher erläutert. Wo Keimzahlreduktionen dargestellt sind, erfolgen diese anhand eines dekadisch-logarithmischen Maßstabs der Ordinate:

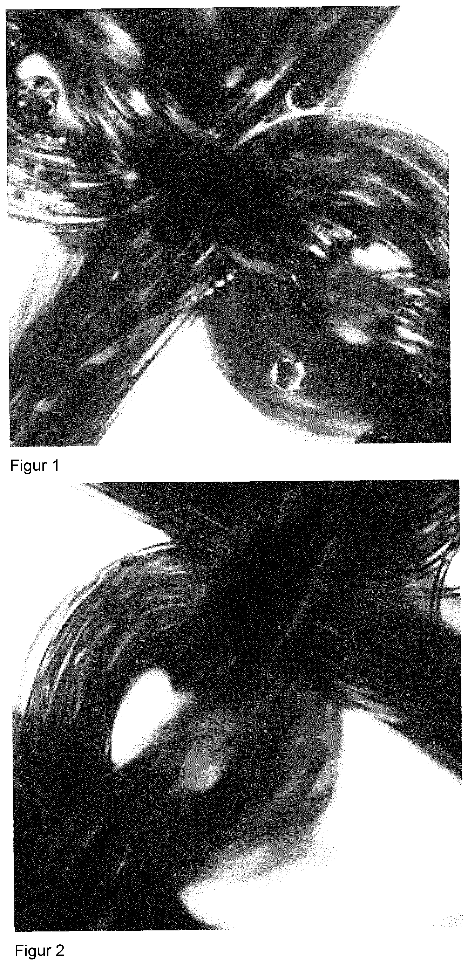

Fig. 1 und Fig. 2 zeigen jeweils eine mittels Konfokalmikroskop gewonnene Aufnahme (40 x) eines im Tauchverfahren beschichteten netzförmigen Substrates in Form eines Polyestergewirkes, wobei das in der Beschichtung enthaltene Polyarginin mit fluoreszierendem FITC gekoppelt war.

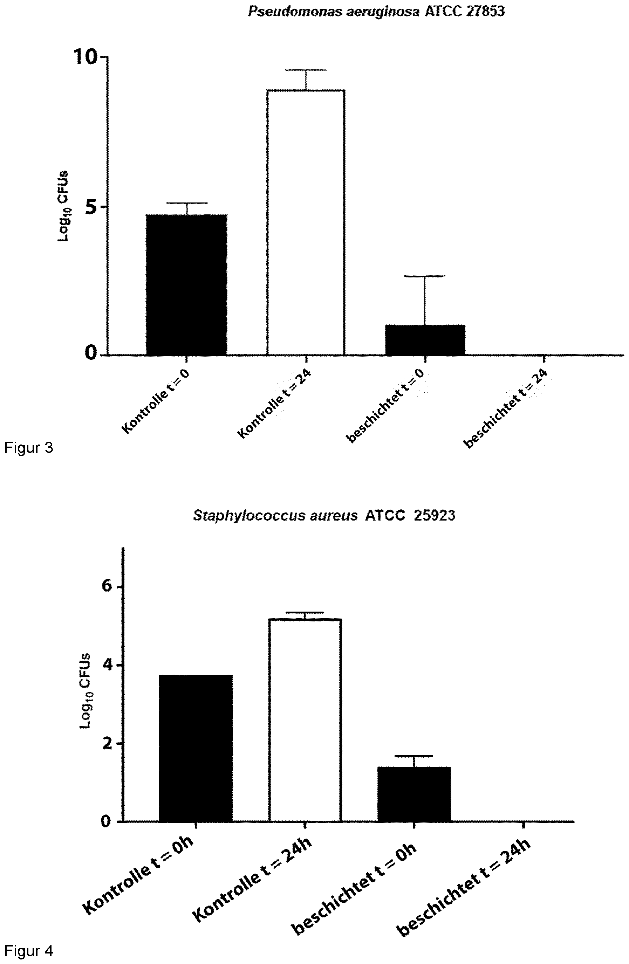

Fig. 3 und Fig. 4 zeigen als Balkendiagramm die Bakterienzahl von *P*. *aeruginosa* (Fig. 3) und *S*. *aureus* (Fig. 4) auf Wundkontaktschichten mit erfindungsgemäßer Tauchbeschichtung (48 alternierende Lagen von PAR30 und HA144) sowie auf identisch aufgebauten Wundkontaktschichten ohne Beschichtung (Kontrolle) für den Initialkontakt (t = 0, dargestellt durch schwarze Balken) sowie für den Zeitpunkt nach vierundzwanzig Stunden (t = 24, dargestellt durch weiße Balken). Die Messwerte sind als Mittelwerte aus drei Wiederholungen dargestellt. Die Varianz der Einzelmessungen wird oberhalb der Balken durch Striche symbolisiert.

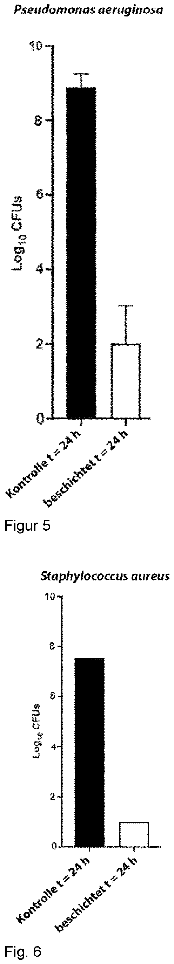

Fig. 5 und Fig. 6 zeigen als Balkendiagramm die Bakterienzahl von *P*. *aeruginosa* (Fig. 5) und *S*. *aureus* (Fig. 6) auf Wundkontaktschichten mit erfindungsgemäßer Sprühbeschichtung (durchmischte Lage aus Polylysin und HA144) sowie auf identisch aufgebauten Wundkontaktschichten ohne Beschichtung (Kontrolle , dargestellt durch schwarzen Balken) für den Zeitpunkt nach vierundzwanzig Stunden (t = 24, dargestellt durch weißen Balken). Die Messwerte sind als Mittelwerte aus drei Wiederholungen dargestellt. Die Varianz der Einzelmessungen wird oberhalb der Balken durch Striche symbolisiert.

### Beispiele

### Beispiel 1: Bereitstellung von Materialien für die Beschichtung eines Substrates

An Materialien wurden bereitgestellt:
- Polypeptide vom Typ Polyarginin als synthetisches Polymer bestehend aus 30 Aminosäuren pro Molekül ("PAR30"), so dass jedes Molekül eine Molekülmasse von etwa 5,8 kDa hatte. Das Polyarginin wurde vom Unternehmen "Alamanda^{™} Polymers" bezogen. Die eingesetzte Konzentration lag bei 0,5 mg / ml.
- Polypeptide vom Typ Polylysin, welche als ε-Poly(L-Lysin) natürlichen Ursprungs (Herstellung mittels Bakterien aus der Familie Streptomycetaceae) vom Unternehmen "Biosynth^{®}" bezogen wurden. Die mittlere Molekülmasse lag zwischen 3,5 und 4,5 kDa. Die eingesetzte Konzentration lag bei 10 mg / ml.
- Hyaluronsäure aus 144 repetitiven Untereinheiten pro Molekül ("HA144"). Hergestellt mittels rekombinanter Produktion aus Mikroorganismen. Die eingesetzte Konzentration lag bei 0,5 mg / ml bis 0,5 mg / ml. Bezogen wurde die Hyaluronsäure vom Unternehmen "Lifecore^{®} Biomedical".
- Netzförmige Polyestersubstrate aus Polyethylenterephthalat in Form eines Tüllgewebes mit einer Dichte von 1,3 - 1,4 g / cm³.

### Beispiel 2: Beschichtung mittels Tauchverfahren

Die Ausgangsmaterialien entsprachen den in Beispiel 1 genannten Spezifikationen. Die bereitgestellten Substrate hatten eine Fläche von 2 cm x 2 cm und wurden zunächst in einem Autoklaven sterilisiert. Während des nachfolgenden Beschichtungsvorgangs mittels Tauchverfahren wurden die Substrate abwechselnd in ein Bad mit Polyarginin ("PAR30" ; 0,5 mg / mL) und ein Bad mit Hyaluronsäure (0,5 mg / mL) eingetaucht. Der erste Eintauchschritt erfolgte dabei in der Lösung mit Polyarginin. Das Eintauchen erfolgte jeweils für 200 Sekunden. Nach jedem Tauchschritt wurde das Substrat gespült, und zwar ebenfalls für 200 Sekunden. Hierzu wurde Tris-NaCl-Puffer verwendet (10 mmol Tris, 150 mmol NaCl, pH 7,4). Der Prozess fand voll automatisiert statt unter Einsatz eines Roboters der Firma "Riegler & Kirstein GmbH". Die genannten Schritte wurden so oft wiederholt, bis 24 Doppellagen auf das Substrat aufgetragen waren. Jede Doppellage bestand aus einer Lage enthaltend das Polyarginin und eine Lage enthaltend die Hyaluronsäure. Somit enthielt die fertige Beschichtung 48 Einzellagen. Der Beschichtungsvorgang dauerte insgesamt etwa 13 Stunden. Bei Bedarf kann der Prozess auch auf eine Dauer von unter zwei Stunden verkürzt werden. Nach einer Trocknung (über Nacht, passiv bei Raumtemperatur) erfolgte im Anschluss eine weitere Sterilisation mittels U.V.-Bestrahlung, und zwar über einen Zeitraum von mindestens 30 min für jeweils die proximale und distale Oberfläche der Wundkontaktschichten.

### Beispiel 3: Beschichtung mittels Sprühverfahren

Die Ausgangsmaterialien entsprachen den in Beispiel 1 genannten Spezifikationen. Es wurden vier Substrate mit einer Fläche von jeweils 2,25 cm² bereitgestellt. Bei der Beschichtung mittels Sprühverfahren kamen eine Lösung enthaltend ε-Poly(L-Lysin) (10 mg / ml) und eine Lösung enthaltend Hyaluronsäure ("HA144" ; 0,5 mg / ml) zum Einsatz, wobei beide Lösungen zeitgleich über jeweils eine separate Düse einer Sprühpistole auf das Substrat aufgesprüht wurden. Das Aufsprühen fand aus einer Distanz von 10 bis 15 cm statt. Dabei wurde die Sprühpistole während des Sprühens zehn Mal für jeweils eine Sekunde über jedes Substrat geführt, um pro Substrat insgesamt 1,5 ml von jeder Lösung aufzutragen. Somit Betrug das Flächenvolumen der Beschichtungslösung 0,66 ml / cm² Substratfläche. Der Beschichtungsvorgang dauerte 10 - 15 Sekunden. Anschließend folgte eine Trocknung (über Nacht, passiv bei Raumtemperatur).

### Beispiel 4: Visuelle Inspektion der Beschichtung mittels Konfokalmikroskop

Die im Tauchverfahren erzeugte Beschichtung wurde einer visuellen Prüfung mittels Konfokalmikroskop unterzogen. Hierzu wurde das in der Beschichtung enthaltene Polyarginin mit dem Fluorophor Fluoresceinisothiocyanat (FITC) markiert. (PAR30-FITC). Die Verteilung der auf diese Wiese zur Fluoreszenz gebrachten Moleküle wurde mit einem Mikroskop vom Typ Zeiss LSM 710 begutachtet. Dies fand bei einer 40-fachen Vergrößerung statt. Die korrekte Anhaftung der Beschichtung an der Tüllstruktur des verwendeten netzförmigen Substrates konnte verifiziert werden. Das Ergebnis ist in den Fig. 1 und 2 als digital nachbearbeitete Fotoaufnahme dargestellt. Wie zu erkennen ist, umschließt die Beschichtung die Faserstruktur des Gewirkes zu allen Seiten auf gleichmäßige Weise. Dies lässt auf eine erfolgreiche Anhaftung der Beschichtung am Substrat schließen.

### Beispiel 5: Antimikrobielle Wirksamkeit der Tauchbeschichtung

Die Tests zur antimikrobiellen Wirksamkeit der beschichteten Wundkontaktschichten wurden sowohl mit gramnegativen als auch mit grampositiven Bakterienstämmen durchgeführt. Als gramnegative Kultur diente ein Stamm von *Pseudomonas aeruginosa* (ATTC 27853) und als grampositive Kultur ein Stamm von *Staphylococcus aureus* (ATTC 25923).

Als Testprotokoll diente der Standard ISO 20743:2021 (Owen L, Laird K. Development of a silver-based dual-function antimicrobial laundry additive and textile coating for the decontamination of healthcare laundry. J Appl Microbiol. 2021;130(4):1012-22). Beim Test wurde die Reduktion der Anzahl teilungsfähiger Bakterienzellen (CFU) auf den textilen Oberflächen nach einer Kontaktzeit von 0 h und 24 h ermittelt. Als Proben dienten netzförmige Substrate aus Polyestertüll. Die antimikrobielle Beschichtung war zuvor im Tauchverfahren auf die Substrate aufgetragen worden und bestand aus einer alternierenden Abfolge von PAR30 und HA144 mit 48 Einzellagen. Weitere Details können dem entsprechenden Ausführungsbeispiel entnommen werden.

Zunächst wurden die Proben mit einer Bakterienausgangskonzentration von 1 bis 3 x 10⁵ CFU /ml beimpft und bei 37 °C inkubiert. Im Anschluss (nach 0 bzw. 24 h) wurden die überlebenden Bakterien in PBS eluiert. Das Eluat wurde abwechselnd gevortext (mit hoher Frequenz mittels eines Vortex Gerätes geschüttelt), sonifiziert und wieder gevortext (jeweils 30 Sek. in dreifacher Wiederholung). Die Proben wurden sowohl im trockenen als auch im feuchten Zustand (nach Zugabe von PBS) getestet, um ein feuchtest Wundmilieu zu simulieren. Als Kontrolle dienten unbeschichtete Wundkontaktschichten mit ansonsten identischem Aufbau. Die Messung wurde insgesamt drei Mal durchgeführt. Die ermittelte antimikrobielle Aktivität ist ausgedrückt als Mittelwert der logarithmischen Reduktion der Anzahl vermehrungsfähiger Bakterien im Vergleich zur Kontrolle. Die Ergebnisse sind dargestellt in den Figuren 3 und 4. Wie aus der Darstellung deutlich wird, setzte bereits nach Initialkontakt mit der Beschichtung (0 h) eine starke antimikrobielle Wirkung ein, deren Effekt nach 24 h noch ausgeprägter war. Die Befeuchtung der Wundkontaktschichten mit PBS hatte keinen Einfluss auf die Ergebnisse.

### Beispiel 6: Antimikrobielle Wirksamkeit der Sprühbeschichtung

Die getesteten Wundkontaktschichten enthielten eine Sprühbeschichtung bestehend aus einer durchmischten Lage enthaltend ε-Poly(L-Lysin) (10 mg / ml) und HA144 (0.5mg / ml). Das Auftragen der Sprühbeschichtung fand gemäß dem entsprechenden Ausführungsbeispiel weiter oben statt. Die antimikrobielle Wirksamkeit der mittels Spühbeschichtung ausgestatteten Wundkontaktschichten wurde mittels ISO 20743:2021 getestet. Die Messung wurde insgesamt drei Mal durchgeführt und die Mittelwerte gebildet. Weitere Details zum Ablauf dieses Tests können dem zuletzt genannten Ausführungsbeispiel entnommen werden, mit dem Unterschied, dass keine Messwerte für den Initialkontakt (t = 0) genommen wurden. Die Ergebnisse sind in Fig. 5 und 6 dargestellt. Wie aus der Darstellung deutlich wird, war nach 24 h ein ausgeprägter antimikrobieller Effekt nachweisbar.

### Beispiel 7: Trocknung von Wundkontaktschichten mit aufgesprühter Beschichtung

Für die oben beschriebenen Ausführungsbeispiele wurden die Proben nach dem Beschichtungsprozess über Nacht bei Raumtemperatur getrocknet.

Zusätzlich fanden antimikrobielle Versuche mit sprühbeschichteten, noch feuchten Proben sowie mit sprühbeschichteten Proben, die 10 min lang bei 80 °C getrocknet wurden statt. Die Sprühbeschichtung wurde gemäß dem entsprechenden Ausführungsbeispiel aufgetragen und enthielt ε-Poly(L-Lysin) sowie HA144. Die ermittelte antimikrobielle Wirkung war in allen Fällen der Sprühbeschichtung identisch (Ergebnisse nicht dargestellt), so dass geschlussfolgert werden kann, dass die antiseptische Wirksamkeit auch bei unterschiedlichem Feuchtigkeitsgehalt der Beschichtung sowie nach aktiver Trocknung erhalten bleibt.

### Beispiel 8: Sterilisation beschichteter Wundkontaktschichten

Es wurden im Tauchverfahren hergestellte Wundkontaktschichten (s. entsprechendes Ausführungsbeispiel oben) bei 120° über einen Zeitraum von 20 Minuten autoklaviert. Die Proben zeigten nach Ablauf der Sterilisation keine erkennbaren Auffälligkeiten. Die anschließende Überprüfung der antimikrobiellen Aktivität (s. entsprechendes Ausführungsbeispiel oben) ergab ebenfalls keine Unterschiede zum vorherigen Test (Ergebnisse nicht dargestellt).

### Beispiel 9: Substrate für Wundkontaktschichten

Bereitgestellt wurden die folgenden Substrate, welche sich zum Einsatz als Wundkontaktschichten eignen und mit der erfindungsgemäßen Beschichtung ausgestattet werden können:
1) Substrat aus Polyamid:

| | |
|---|---|
| Faseranordnung: | Gewirke in Form eines Tülls |
| Material: | Nylon 6.6 |
| Dicke: | 0,22 mm |
| Fläche: | 10 cm x 10 cm |

2) Substrat aus Polyethylenterephthalat:

| | | |
|---|---|---|
| Faseranordnung: | Gewirke | |
| Lineare Dichte: | 76 dtex | (DIN EN ISO 2060) |
| Bruchdehnung: | 26 % | (DIN EN ISO 2062) |
| Festigkeit: | 37 cN/tex | (DIN EN ISO 2062) |
| Dichte: | 1,3 - 1,4 g / cm³ | |
| Flächengewicht: | 85 g / m² | |

3) Substrat mit elementarem, nicht ionischem Silber in der Faserstruktur:

| | |
|---|---|
| Faseranordnung: | Gewirke in Form eines Tülls |
| Material: | 90% Polyamid in Form von Nylon 6.6 und 10 % elementares Silber |
| Dicke: | 0,22 mm |
| Fläche: | 10 cm x 10 cm |
| Flächengewicht: | 33 g / m² |

## Patentansprüche

1. Wundkontaktschicht umfassend ein netzförmiges Substrat, welches teilweise oder vollständig eine antimikrobielle Beschichtung aufweist, welche Hyaluronsäure und Polypeptide ausgewählt aus Polyarginin oder Polylysin oder einer Mischung aus Polyarginin und Polylysin umfasst.

2. Wundkontaktschicht nach Anspruch 1, wobei die Polypeptide mindestens zehn Aminosäuren und/oder höchstens hundert Aminosäuren umfassen.

3. Wundkontaktschicht nach einem der vorhergehenden Ansprüche, wobei die Hyaluronsäure als Polymermischung mit unterschiedlicher Kettenlänge vorliegt und wobei die Polymermischung Polymere mit einem Molekulargewicht von mindestens 10 kDa umfasst.

4. Wundkontaktschicht nach einem der vorhergehenden Ansprüche, wobei die Hyaluronsäure als Polymermischung mit unterschiedlicher Kettenlänge vorliegt und wobei die in der Polymermischung enthaltenen Polymere ein Molekulargewicht von höchstens 300 kDa aufweisen.

5. Wundkontaktschicht nach einem der vorhergehenden Ansprüche, wobei die Polypeptide und die Hyaluronsäure innerhalb der Beschichtung durchmischt vorliegen und die Polypeptide in einer Hyaluronsäurematrix eingebettet sind.

6. Wundkontaktschicht nach einem der vorhergehenden Ansprüche 1 bis 4, wobei die Beschichtung mindestens zwei übereinanderliegende und miteinander verbundenen Lagen enthält,
und wobei mindestens eine Lage vorhanden ist, welche die Polypeptide enthält und eine positive Nettoladung hat, sowie mindestens eine Lage, welche die Hyaluronsäure enthält und eine negative Nettoladung hat,
und wobei sich bei den übereinanderliegenden Lagen jeweils eine die Polypeptide enthaltende Lage mit einer die Hyaluronsäure enthaltenden Lage abwechselt, so dass eine Abfolge von alternierenden Lagen gebildet wird.

7. Wundkontaktschicht nach Anspruch 6, wobei die Anzahl der übereinanderliegenden und miteinander verbundenen Lagen in der Beschichtung 10 bis 100 beträgt.

8. Wundkontaktschicht nach einem der vorhergehenden Ansprüche, wobei die Polypeptide ein Molekulargewicht von 1 bis 41 kDa haben.

9. Wundkontaktschicht nach einem der vorhergehenden Ansprüche, wobei die Beschichtung eine Dicke von 10 nm bis 1000 nm hat.

10. Wundkontaktschicht nach einem der vorhergehenden Ansprüche, wobei die Beschichtung trocknungsbeständig ist.

11. Wundkontaktschicht nach einem der vorhergehenden Ansprüche, wobei das netzförmige Substrat mindestens eine der folgenden Fasern enthält oder daraus besteht:
Polyamidfasern, insbesondere Nylonfasern, Polyesterfasern, Baumwollfasern, Viskosefasern.

12. Wundkontaktschicht nach einem der vorhergehenden Ansprüche, wobei das netzförmige Substrat ein Gewirke oder Tüllgewebe ist.

13. Wundkontaktschicht nach einem der vorhergehenden Ansprüche, wobei das netzförmige Substrat ein Tüllgewebe ist, das Polyester oder Polyamid enthält.

14. Verfahren zur Herstellung einer Wundkontaktschicht nach einem der Ansprüche 1 bis 13 umfassend die folgenden Schritte:
i) Bereitstellung eines netzförmigen Substrates
ii) Besprühen des netzförmigen Substrates mit Hyaluronsäure und Polypeptiden zur Ausbildung einer Beschichtung umfassend mindestens eine Lage,
wobei es sich bei den Polypeptiden um Polyarginin und / oder Polylysin handelt und wobei die mindestens eine Lage sowohl die Hyaluronsäure als auch die Polypeptide enthält.

15. Verfahren nach Anspruch 14, wobei die Hyaluronsäure und die Polypeptide während des Besprühens in Lösung vorliegen.
